# FASCICULE DE BREVET EUROPEEN

(11) **EP 1 689 343 B1**
(45) Date de publication et mention de la délivrance du brevet: **11.07.2012**
(21) Numéro de dépôt: 04805633.7
(22) Date de dépôt: 03.12.2004
(51) Int. Cl.: A61J 15/00, A61M 39/10

(54) **Raccords mâles et raccords femelles pour réaliser des connexions de transmission de liquide, notamment pour des lignes de nutrition entérale**
Männliche Verbindungsglieder und weibliche Verbindungsglieder zur Herstellung von Flüssigkeitsüberleitungsverbindungen wie z.b. für enterale Ernährungsleitungen
Male connectors and female connectors which are used to produce liquid transmission connections, such as for enteral nutrition lines

(30) Priorité: 05.12.2003 FR 0314292
(43) Date de publication de la demande: 16.08.2006
(73) Titulaire: Vygon, 95440 Ecouen (FR)
(72) Inventeur: CARREZ, Jean-Luc, F-95440 Ecouen (FR); DALLE, Valéry, F-60270 Gouvieux (FR); GUYOMARC'H, Pierrick, F-95120 Ermont (FR); TEMPEREAU, Michel, F-95270 Luzarches (FR)
(74) Mandataire: Texier, Christian
(86) Numéro de dépôt international: PCT/FR2004/003114
(87) Numéro de publication internationale: WO 2005/055919

(56) Documents cités:
- WO-A-01/83001
- GB-A- 2 383 828
- US-A- 3 751 077
- US-A- 5 971 972

## Description

L'invention concerne des raccords pour assemblage conique et à filetage de verrouillage, utilisables pour réaliser des connexions de transmission de liquide dans le domaine de l'appareillage médical, notamment pour des lignes de nutrition entérale.

Une ligne de nutrition entérale comprend généralement un conteneur de nutriment relié par une tubulure flexible à une sonde de nutrition entérale.

Ces trois composants doivent être raccordés de proche en proche.

Habituellement, le conteneur présente une extrémité de raccordement qui constitue ou qui est munie d'un raccord mâle et la sonde de nutrition présente une extrémité de raccordement qui constitue ou qui est munie d'un raccord femelle.

Le raccord mâle du conteneur peut être raccordé directement au raccord femelle de la sonde mais le plus souvent ce raccordement se fait par l'intermédiaire d'une tubulure flexible qui se termine à une extrémité par un raccord femelle apte à se raccorder au raccord mâle du conteneur et qui se termine à son extrémité opposée par un raccord mâle apte à se raccorder au raccord femelle de la sonde.

Des raccords sont également utilisés dans d'autres dispositifs médicaux, par exemple dans des dispositifs de perfusion, pour des cathéters intraveineux ou artériels, etc.

Des normes ont été établies pour l'ensemble de ces raccords, leur imposant certaines dimensions.

La norme NF EN 20 594 concerne des raccords conçus pour réaliser des assemblages coniques avec une conicité imposée à 6% (Luer) et définit notamment les diamètres d'entrée minimal et maximal des raccords.

Selon la norme NF EN 20 594, les raccords mâles pour assemblages coniques Luer ont un diamètre d'entrée compris dans la gamme 3,925 mm - 3,990 mm (matériau rigide) ou dans la gamme 3,925 mm - 4,027 mm (matériau semi-rigide), et les raccords femelles ont un diamètre d'entrée compris dans la gamme 4,270 mm - 4, 315 mm.

Les diamètres d'entrée sont les diamètres qui conditionnent la possibilité d'emboîter le raccord mâle dans le raccord femelle : il s'agit donc du diamètre extérieur dans le cas du raccord mâle et du diamètre intérieur dans le cas du raccord femelle.

La norme NF EN 1707 définit des raccords pour assemblage conique munis de filetages de verrouillage aptes à coopérer pour réaliser le verrouillage de la connexion et définit notamment les diamètres à la base des filets et au sommet des filets de ces raccords.

Selon la norme NF 1 707, les filets des raccords coniques Luer répondent aux conditions suivantes :
- pour le raccord mâle, diamètre du filet à la base 8,00 mm et diamètre du filet au sommet 7,00 mm ;
- pour le raccord femelle, diamètre du filet à la base 6,73 mm maximum et diamètre du filet au sommet 7,83 mm.

Les raccords femelles normalisés présentent une tête qui détermine le conduit d'entrée du raccord et dans la face extérieure de laquelle est formée le filetage si bien que le diamètre extérieur de cette tête est aussi le diamètre des filets au sommet, tandis que les raccords mâles normalisés présentent un embout en saillie qui détermine le conduit d'entrée du raccord mâle et qui est entouré d'une collerette fixe ou mobile, laquelle détermine autour de l'embout une gorge pour recevoir la tête d'un raccord femelle correspondant et sur la face intérieure de laquelle est formée le filetage du raccord si bien que le diamètre au sommet des filets du raccord mâle délimite le diamètre de passage de la gorge du raccord.

Pour l'assemblage, la tête de l'embout mâle est apte à pénétrer avec étanchéité latérale dans le conduit d'entrée du raccord femelle, la tête de l'embout femelle est apte à pénétrer dans la gorge du raccord mâle, et les filetages des deux raccords étant aptes à coopérer pour verrouiller l'assemblage.

Le fait que ces normes s'appliquent indifféremment aux raccords pour nutrition entérale et aux raccords pour perfusion veineuse est une cause potentielle d'accident.

Il peut arriver en effet que le conteneur de nutriment par exemple, une seringue, soit utilisée accidentellement pour alimenter une ligne de perfusion ou un cathéter veineux ou artériel, et il peut arriver qu'un conteneur contenant un produit autre qu'un nutriment soit utilisé pour alimenter une sonde pour nutrition entérale.

Différentes mesures ont été préconisées pour éviter ces raccordements indésirables.

On a pensé à distinguer les raccords par des codes de couleurs, ce qui est une précaution insuffisante.

En ce qui concerne les seringues, on a proposé (brevet FR 787 999) de les munir d'une extrémité luer femelle au lieu de l'extrémité mâle luer habituelle.

On a également proposé (brevet FR 2 801 987) d'utiliser des connecteurs mâles non luer muni d'un bourrelet d'extrémité.

La publication WO 01/83001 décrit des raccords médicaux ayant des diamètres non conventionnels conçus pour pouvoir s'accoupler entre eux ou s'accoupler avec des raccords conventionnels par l'intermédiaire d'adaptateurs.

De fait cette publication vise à pouvoir utiliser des raccords ayant des diamètres internes supérieurs à ceux des raccords conventionnels et à pouvoir les raccorder néanmoins, si nécessaire, à des raccords conventionnels au moyen d'adaptateurs.

La publication US 3 751 077 décrit des raccords métalliques pour haute pression ou tension élevée.

La publication GB 2 383 828 vise à empêcher des erreurs de raccordements dans le domaine médical et elle préconise à cette fin des raccords qu'elle qualifie de « différents », conçus pour ne pas pouvoir se raccorder à des raccords standards.

Selon cette publication, on peut réaliser cette différence en donnant au raccord mâle une conicité différente de 6% en sorte que ce raccord ne s'apparie pas à un raccord femelle standard de conicité luer, c'est-à-dire ayant une conicité de 6 %.

Une simple différence de conicité n'empêche pas en soi l'introduction du raccord mâle différent dans le raccord femelle standard en sorte qu'une erreur de raccordement reste possible même si le raccordement n'est pas parfait.

Une autre différence préconisée dans cette publication réside dans une diminution du diamètre du raccord mâle. Il est clair que cette diminution, si elle peut empêcher le raccordement parfait, n'empêche pas, bien au contraire, l'introduction du raccord mâle diminué dans le raccord femelle standard, en sorte que le risque d'une erreur de raccordement subsiste.

La publication GB 2 383 828 préconise enfin de réaliser un désaccord entre les filetages réciproques de la couronne du raccord mâle et du raccord femelle. Ce désaccord ne signifie pas que le raccord mâle ne puisse pas être introduit dans le raccord femelle, en sorte que le risque d'un mauvais raccordement subsiste.

De fait, la publication GB 2 383 828 vise essentiellement à empêcher un verrouillage d'un raccord différent et d'un raccord standard, mais le risque subsiste d'un raccordement imparfait mais suffisant pour tromper le manipulateur.

La présente invention vise à empêcher toute erreur de manipulation et non pas seulement un verrouillage.

On y parvient selon l'invention avec des raccords mâles et femelles pour assemblages coniques et à filetages de verrouillage, qui se distinguent des raccords normalisés par le fait qu'ils présentent un diamètre d'entrée et un diamètre au sommet des filets choisis par rapport aux diamètres correspondants des raccords normalisés en sorte que l'assemblage d'un raccord mâle (RMI) ou femelle (RFI) selon l'invention, respectivement avec un raccord femelle (RFN) ou mâle (RMN) normalisé, soit empêché parce que la pénétration de l'embout du raccord mâle dans le conduit d'entrée du raccord femelle est impossible ou parce que cette pénétration est arrêtée par butée de la tête du raccord femelle contre la collerette du raccord mâle.

Par exemple, les raccords de l'invention ont les dimensions caractéristiques indiquées dans le tableau ci-après :

| **Diamètres (mm)** | **RMI** | **RFN** | **RFI** | **RMN** |
|---|---|---|---|---|
| d'entrée | 3,2 | 4,270-4,315 | 3,5 | 3,925-4,027 |
| A la base des filets | 7 | 6,73 | 5,6 | 8 |
| Au sommet des filets | 5,8 | 7,83 | 6,8 | 7 |
| d'entrée | 4,5 | 4, 270-4, 315 | 4,8 | 3, 925-4, 027 |
| A la base des filets | 8,4 | 6,73 | 7,2 | 8 |
| Au sommet des filets | 7,4 | 7,83 | 8,2 | 7 |

L'embout d'un raccord mâle selon l'invention (RMI) ayant un diamètre d'entrée de 3,2 mm et un diamètre au sommet des filets de 5,8 mm pourra pénétrer dans le conduit d'entrée d'un raccord femelle normalisé (RFN) mais cette pénétration sera arrêtée par butée de la tête du raccord femelle contre la collerette du raccord mâle, et un raccord femelle selon l'invention (RFI) ayant un diamètre d'entrée de 4,8 mm et un diamètre au sommet des filets de 8,2 mm pourra se laisser pénétrer par l'embout d'un raccord mâle normalisé mais que cette pénétration sera arrêtée par butée de la tête du raccord femelle contre la collerette du raccord mâle.

Au surplus les raccords coniques selon l'invention ont de préférence une conicité différente de la conicité Luer, par exemple une conicité de 4% ou de 8-10%.

Dans des réalisations particulières, les raccords de l'invention présentent encore les caractéristiques suivantes, combinées ou non :
- un cône mâle non luer à 8% (au lieu de 6%), soit une pente de 2°17'26" (au lieu de 1°43'6") ;
- un cône femelle non luer à 8% ;
- une longueur de cône mâle ou femelle de 6,5 mm ;
- un verrouillage à vis à double filet au pas de 5 mm.

Les figures du dessin joint illustrent des exemples A ou C d'un raccord mâle et d'un raccord femelle B ou D conformes à l'invention et aptes à être accouplés :
- la figure 1 est une coupe axiale d'un raccord mâle et du raccord femelle correspondant, selon l'invention ;
- la figure 2 est une coupe axiale des raccords de la figure 1 ;
- la figure 3 montre en coupe axiale (fig.3 (A)) et en perspective (fig.3 (B)) l'assemblage des raccords de la figure 1 ;
- les figures 4 à 6 sont des coupes axiales et les perspectives correspondantes illustrant les impossibilités d'accouplement d'un raccord mâle ou femelle selon la figure 1 avec des raccords normalisés ;
- la figure 7 est une vue en perspective d'un autre raccord mâle et du raccord femelle correspondant, selon l'invention ;
- la figure 8 est une coupe axiale des raccords de la figure 7.
- la figure 9 montre en coupe axiale (fig.9 (A)) et en perspective (fig.9 (B)) l'assemblage des raccords de la figure 6, et
- les figures 10 à 12 sont des coupes axiales et les perspectives correspondantes illustrant les impossibilités d'accouplement d'un raccord mâle ou femelle selon la figure 6 avec des raccords normalisés.

Les raccords mâles A et C selon l'invention présentent de façon en soi connue un embout d'extrémité en saillie (1 ; 2) qui détermine un passage d'entrée conique (3 ; 4) communiquant avec un canal arrière (5 ; 6) et présentent autour de l'embout une collerette (7 ; 8) qui détermine autour de l'embout une gorge (9 ; 10) et la collerette présente un filetage interne (11 ; 12) tourné vers l'embout.

Les raccords femelles B et D selon l'invention présentent de façon en soi connue une tête avant (13 ; 14) qui détermine un conduit d'entrée conique (15 ; 16) qui communique avec un canal arrière (17 ; 18), et la tête est pourvue d'un filetage externe (19 ; 20).

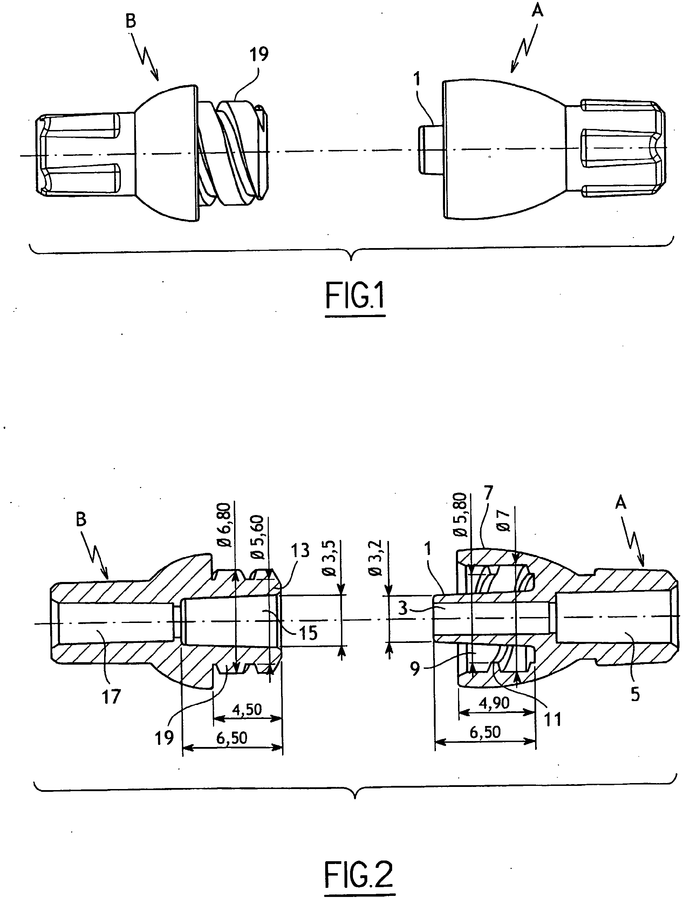

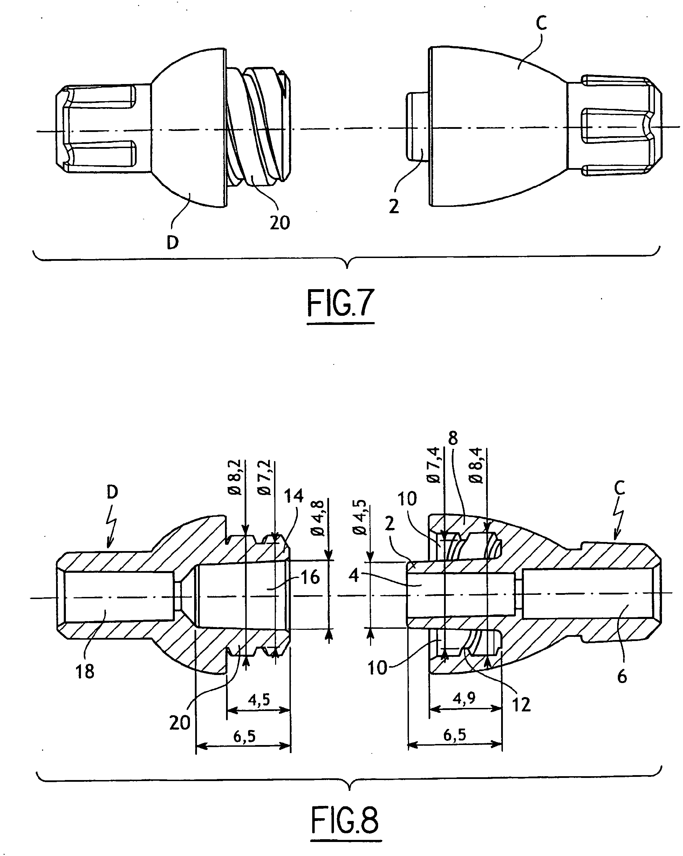

Les dimensions caractéristiques des raccords sont portées sur les figures 1 et 7.

La tête de l'embout femelle B est apte à pénétrer dans la gorge de l'embout mâle A et à s'y visser tandis que la tête de l'embout femelle D est apte à pénétrer dans la gorge de l'embout mâle C et à s'y visser.

L'embout mâle A est apte à pénétrer avec étanchéité latérale dans le conduit d'entrée du raccord femelle B et l'embout mâle C est apte à pénétrer avec étanchéité latérale dans le conduit d'entrée du raccord femelle D.

Les canaux arrières des raccords mâles et des raccords femelles permettent de fixer les raccords à des tubulures, de façon en soi connue.

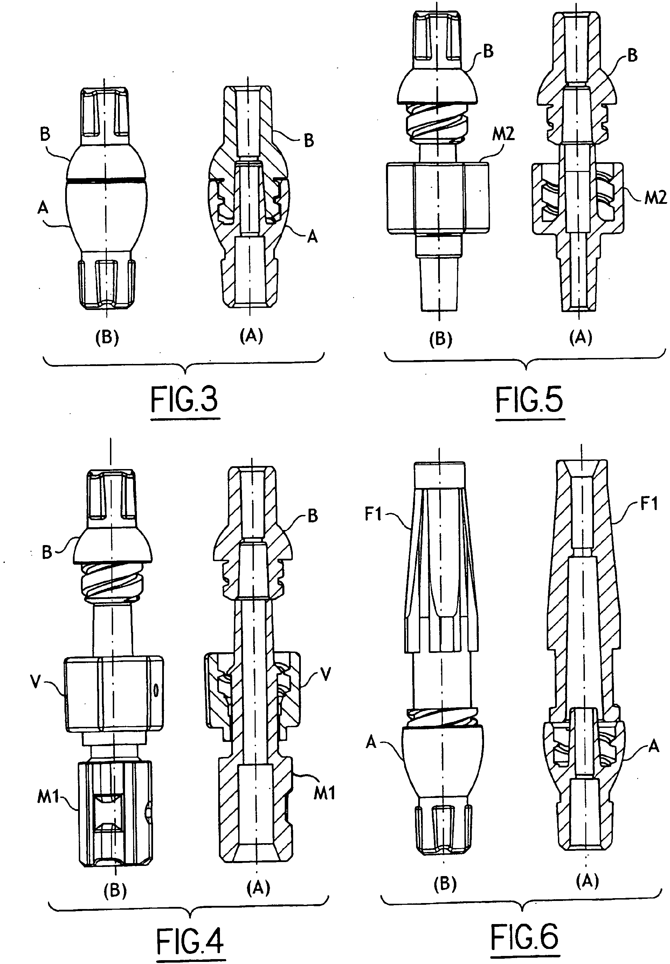

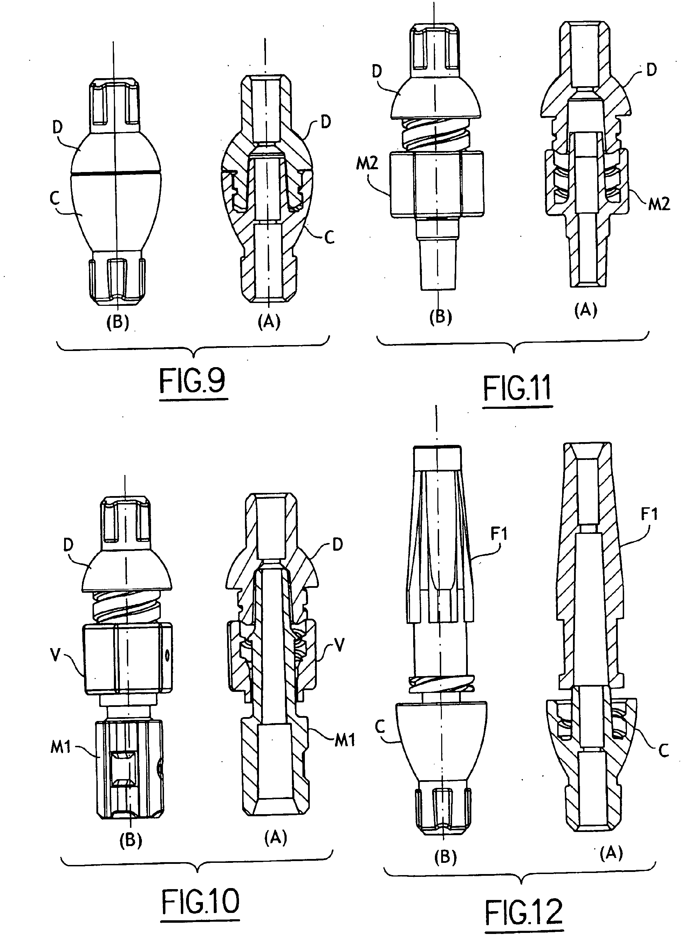

Les dimensions des raccords mâles A et C et des raccords femelles B et D sont choisis pour permettre l'assemblage des raccords A et B comme le montre la figure 3 et l'assemblage des raccords C et D comme le montre la figure 9.

Par contre, ces raccords ne peuvent être assemblés avec des raccords normalisés, comme le montrent pour l'exemple les figures 4 à 6 et 10 à 12 :
- figure 4 : l'embout du raccord mâle normalisé M1 à verrou de blocage coulissant V ne peut rentrer dans le conduit d'entrée du raccord femelle B de l'invention ;
- figure 5 l'embout du raccord mâle normalisé M2 à collerette fixe ne peut rentrer dans le conduit d'entrée du raccord femelle B de l'invention ;
- figure 6 : l'embout du raccord mâle A de l'invention A peut rentrer dans le conduit d'entrée du raccord femelle normalisé F1 mais la tête du raccord femelle ne peut rentrer dans la gorge du raccord mâle ;
- figure 10 : l'embout du raccord mâle normalisé M1 à verrou de blocage coulissant V peut rentrer dans le conduit d'entrée du raccord femelle D selon l'invention mais la tête de ce raccord femelle ne peut rentrer dans la gorge du verrou ;
- figure 11 : l'embout du raccord mâle normalisé M2 peut rentrer dans le conduit d'entrée du raccord femelle D de l'invention mais la tête de ce raccord femelle ne peut rentrer dans la gorge du raccord mâle ;
- figure 12 : l'embout du raccord mâle C de l'invention ne peut rentrer dans le conduit d'entrée du raccord femelle F1 normalisé.

Ces exemples d'incompatibilité ne sont pas limitatifs.

Les raccords mâles (RMI) et femelles (RFI) de l'invention sont destinés notamment à équiper des conteneurs divers (seringues, seringues munies d'une paille, gaveuse, poche, flacon, bouteille) des sondes, des tubulures, des raccords à trois voies.

De ce fait, l'invention a également pour objets :
- un conteneur (notamment poche, flacon, bouteille, seringue, gaveuse) équipé d'un raccord femelle (RFI) ;
- une seringue munie d'une paille pour l'aspiration de produits de nutrition entérale, équipée d'un raccord mâle (RMI) ;
- une sonde qui présente une extrémité de raccordement constituée ou munie d'un raccord femelle (RFI) ;
- une tubulure qui présente une extrémité munie d'un raccord mâle (RMI) ;
- un raccord qui présente une extrémité constituée par un raccord femelle (RFI) et une extrémité opposée apte à se connecter sur un conteneur d'alimentation entérale ;
- un raccord à trois voies dont deux voies sont équipées respectivement d'un raccord mâle (RMI) et d'un raccord femelle (RFI), la troisième voie étant équipée d'un raccord mâle (RMI) ou d'un raccord femelle (RFI) ;
- des ensembles de raccords comprenant des raccords mâles (RMI), des raccords femelles (RFI) et des raccords normalisés, tels que définis dans le tableau.

L'invention n'est pas limitée aux modes de réalisation qui ont été décrits.

## Revendications

1. Raccord mâle (RMI) et raccord femelle (RFI) à assembler et à verrouiller entre eux pour réaliser une connexion de transmission de liquide dans le domaine de l'appareillage médical où l'on utilise couramment des raccords mâles (RMN) et des raccords femelles (RFN) normalisés, notamment pour une ligne de nutrition entérale, le raccord femelle (RFI) présentant comme le raccord femelle normalisé (RFN) une tête (13 ;14) qui détermine un conduit d'entrée conique (15 ;16) et qui présente un filetage externe (19 ;20), et le raccord mâle (RMI) présentant comme le raccord mâle normalisé (RMN) un embout conique (1 ;2) en saillie qui détermine un conduit d'entrée (3 ;4) entouré d'une collerette (7 ;8), qui détermine autour de l'embout une gorge (9 ;10) et qui présente un filetage interne (11,12), la tête de l'embout femelle (RFI) étant apte à pénétrer dans la gorge du raccord mâle (RMI) la tête de l'embout mâle (RMI) étant apte à pénétrer avec étanchéité latérale dans le conduit d'entrée du raccord femelle (RFI), et les filetages des deux raccords étant aptes à coopérer pour verrouiller l'assemblage, **caractérisés en ce que** ces raccords (RMI, RFI) présentent un diamètre d'entrée et un diamètre au sommet des filets choisis par rapport aux diamètres correspondants des raccords normalisés (RMN, RFN), en sorte que l'assemblage d'un raccord mâle (RMI) ou femelle (RFI), respectivement avec un raccord femelle (RFN) ou mâle (RMN) normalisé, soit empêché parce que la pénétration de l'embout du raccord mâle dans le conduit d'entrée du raccord femelle est impossible ou parce que cette pénétration est arrêtée par butée de la tête du raccord femelle contre la collerette du raccord mâle.

2. Raccords (RMI, RFI) selon la revendication 1 dont les conduits d'entrée (15;16- 3;4) ont une conicité différente de 6% (conicité luer)

3. Raccords (RMI, RFI) selon la revendication 2 dont les conduits d'entrée (15;16- 3;4) ont une conicité comprise dans la gamme 4% et 8-10%.

4. Raccords (RMI, RFI) selon l'une des revendications 1 à 3 dont les conduits d'entrée (15;16-3;4) coniques ont une longueur de 6,5 mm.

5. Raccords (RMI, RFI) selon l'une des revendications 1 à 4 dont les filetages sont à double filets au pas de 5 mm.

6. Raccords (RMI, RFI) selon l'une des revendications 1 à 5 dont le raccord mâle (A) a un diamètre d'entrée de 3,2 mm et un diamètre au sommet des filets de 5,8 mm et dont le raccord femelle (B) a un diamètre d'entrée de 3,5 mm et un diamètre au sommet des filets de 6,8 mm.

7. Raccords (RMI, RFI) selon la revendication 6 dont le raccord mâle (A) a un diamètre à la base des filets de 7 mm.

8. Raccords (RMI, RFI) selon la revendication 6 dont le raccord femelle (B) a un diamètre à la base des filets de 5,6 mm.

9. Raccords (RMI, RFI) selon l'une des revendications 1 à 5 dont le raccord mâle (C) a un diamètre d'entrée de 4,5 mm et un diamètre au sommet des filets de 7,4 mm et dont le raccord femelle (D) a un diamètre d'entrée de 4,8 mm et un diamètre au sommet des filets de 8,2 mm.

10. Raccords (RMI, RFI) selon la revendication 9 dont le raccord mâle (C) a un diamètre à la base des filets de 8,4 mm.

11. Raccords (RMI, RFI) selon la revendication 12 dont le raccord femelle (D) a un diamètre à la base des filets de 7,2 mm.

12. Raccords (RMI, RFI) selon l'une des revendications 1 à 11 qui présentent des canaux arrières (5,6 ; 17,18) permettant de fixer les raccords à des tubulures.

13. Conteneur équipé d'un raccord femelle (RFI) tel que défini dans l'une des revendications 1 à 11.

14. Conteneur selon la revendication 13 du groupe constitué par une poche, un flacon, une bouteille, une seringue.

15. Gaveuse de nutrition entérale équipée d'un raccord femelle (RFI) tel que défini dans l'une des revendications 1 à 11.

16. Seringue munie d'une paille pour l'aspiration de produits de nutrition entérale équipée d'un raccord mâle (RMI) tel que défini dans l'une des revendications 1 à 11.

17. Sonde qui présente une extrémité de raccordement constituée ou munie d'un raccord femelle (RFI) tel que défini dans l'une des revendications 1 à 11.

18. Tubulure qui présente une extrémité munie d'un raccord mâle (RMI) selon l'une des revendications 1 à 11 et une extrémité opposée munie d'un raccord femelle (RFI) selon l'une des revendications 1 à 11.

19. Raccord qui présente une extrémité constituée par un raccord femelle (RFI) selon l'une des revendications 1 à 11 et une autre extrémité apte à se connecter sur un conteneur d'alimentation entérale.

20. Raccord à trois voies dont une voie est équipée d'un raccord mâle (RMI) selon l'une des revendications 1 à 11, les deux autres voies étant équipées chacune d'un raccord femelle (RFI) selon l'une des revendications 1 à 11.

21. Raccord à trois voies dont une voie est équipée d'un raccord femelle (RFI) selon l'une des revendications 1 à 11, les deux autres voies étant équipées chacune d'un raccord mâle (RMI) selon l'une des revendications 1 à 11.

22. Lignes de nutrition entérale munies d'un raccord mâle (RMI) et d'un raccord femelle (RFI) selon une ou plusieurs des revendications 1 à 11.

23. Ensemble de raccords comprenant des raccords normalisés (RMN, RFN) et des raccords selon l'invention (RMI, RFI) tels que définis dans le tableau suivant :
| **Diamètres (mm)** | **RMI** | **RFN** | **RFI** | **RMN** |
|---|---|---|---|---|
| d'entrée | 3,2 | 4,270-4,315 | 3,5 | 3,925-4,027 |
| A la base des filets | 7 | 6,73 | 5,6 | 8 |
| Au sommet des filets | 5,8 | 7, 83 | 6,8 | 7 |
| d'entrée | 4,5 | 4,270-4,315 | 4,8 | 3,925-4,027 |
| A la base des filets | 8,4 | 6,73 | 7,2 | 8 |
| Au sommet des filets | 7,4 | 7,83 | 8,2 | 7 |

## Claims

1. A male connector (RMI) and a female connector (RFI) to be assembled and to be locked together in order to create a liquid transmission connection in the field of medical coupling, in which standardised male connectors (RMN) and female connectors (RFN) are currently used, in particular for an enteral nutrition line, with the female connector (RFI) having as the standardised female connector (RFN) a head (13 ; 14) that forms a conical entry conduit (15 ; 16) and which has an external thread (19 ; 20), and with the male connector (RMI) having as standardised male connector (RMN) a projecting conical tube (1 ; 2) that forms an entry conduit (3 ; 4), surrounded by a collar (7 ; 8) which forms a channel (9 ; 10) around the tube, and which has an internal thread (11 ; 12), with the head of the female tube (RFI) being capable of penetrating into the channel of the male connector (RMI), with the head of the male tube (RMI) being capable of penetrating, with lateral sealing, into the entry conduit of the female connector (RFI), and with the threads of the two connectors being capable of fitting together so as to lock the assembly, **characterised in that** these connectors (RMI, RFI) have an entry diameter and a diameter at the crest of the threads that are chosen in relation to the corresponding diameters of the standardised connectors (RMN, RFN), so that the assembly of a male connector (RMI) or female connector (RFI) with a standardised female connector (RFN) or standardised male connector (RMN) respectively, is prevented because penetration of the ferrule of the male connector into the entry conduit of the female connector is impossible or because this penetration is halted by the head of the female connector butting against the collar of the male connector.

2. Connectors (RMI, RFI) according to claim 1, in which the entry conduits (15 ; 16 - 3 ; 4) have a taper other than 6% (the Luer taper).

3. Connectors (RMI, RFI) according to claim 2, in which the entry conduits (15 ; 16 - 3 ; 4) have a taper falling within the range 4% and 8 - 10%.

4. Connectors (RMI, RFI) according to one of claims 1 to 3, in which the conical entry conduits (15 ; 16 - 3 ; 4) have a length of 6.5 mm.

5. Connectors (RMI, RFI) according to one of claims 1 to 4, in which the threads are double threads with a pitch of 5 mm.

6. Connectors (RMI, RFI) according to one of claims 1 to 5, in which the male connector (A) has an entry diameter of 3.2 mm and a diameter at the crest of the threads of 5.8 mm and in which the female connector (B) has an entry diameter of 3.5 mm and a diameter at the crest of the threads of 6.8 mm.

7. Connectors (RMI, RFI) according to claim 6, in which the male connector (A) has a diameter of 7 mm at the base of the threads.

8. Connectors (RMI, RFI) according to claim 6, in which the female connector (B) has a diameter of 5.6 mm at the base of the threads.

9. Connectors (RMI, RFI) according to one of claims 1 to 5, in which the male connector (C) has an entry diameter of 4.5 mm and a diameter at the crest of the threads of 7.4 mm, and in which the female connector (D) has an entry diameter of 4.8 mm and a diameter at the crest of the threads of 8.2 mm.

10. Connectors (RMI, RFI) according to claim 9, in which the male connector (C) has a diameter of 8.4 mm at the base of the threads.

11. Connectors (RMI, RFI) according to claim 12, in which the female connector (D) has a diameter of 7.2 mm at the base of the threads.

12. Connectors (RMI, RFI) according to one of claims 1 to 11, which have rear channels (5 ; 6, 17 ; 18) that allow attachment of the connectors to tubes.

13. A container fitted with a female connector (RFI) as described in one of claims 1 to 11.

14. A container according to claim 13, from the group composed of a sachet, a flask, a bottle, or a syringe.

15. An enteral nutrition force-feeder, fitted with a female connector (RFI) as described in one of claims 1 to 11.

16. A syringe equipped with a pipette for the take-up of enteral nutrition products, fitted with a male connector (RMI) as described in one of claims 1 to 11.

17. A probe which has a connecting end composed of or equipped with a female connector (RFI) as described in one of claims 1 to 11.

18. A tube which has one end equipped with a male connector (RMI) according to one of claims 1 to 11, and an opposite end equipped with a female connector (RFI) according to one of claims 1 to 11.

19. A connector which has one end composed of a female connector (RFI) according to one of claims 1 to 11, and another end which is capable of connecting to an enteral feed container.

20. A three-way connector in which one channel is fitted with a male connector (RMI) according to one of claims 1 to 11, with each of the other two channels being equipped with a female connector (RFI) according to one of claims 1 to 11.

21. A three-way connector in which one channel is fitted with a female connector (RFI) according to one of claims 1 to 11, with each of the other two channels being equipped with a male connector (RMI) according to one of claims 1 to 11.

22. Enteral nutrition lines fitted with a male connector (RMI) and a female connector (RFI) according to one or more of claims 1 to 11.

23. A set of connectors that include the standardised connectors (RMN, RFN) as specified in the table, and also connectors according to the invention (RMI, RFI) as specified in the table.
| DIAMETER (MM) | RMI | RFN | RFI | RMN |
|---|---|---|---|---|
| Entry | 3.2 | 4.270-4.315 | 3.5 | 3.925-4.027 |
| At the base of the threads | 7 | 6.73 | 5.6 | 8 |
| At the crest of the threads | 5.8 | 7.83 | 6.8 | 7 |
| Entry | 4.5 | 4.270-4.315 | 4.8 | 3.925-4.027 |
| At the base of the threads | 8.4 | 6.73 | 7.2 | 8 |
| At the crest of the threads | 7.4 | 7.83 | 8.2 | 7 |

## Patentansprüche

1. Steckverbindungsstück (RMI) und Gegensteckverbindungsstück (RFI) zum gegenseitigen Zusammenbau und Verriegeln, um eine Flüssigkeitsüberleitungsverbindung auf dem Gebiet medizinischer Vorrichtungen zu realisieren, bei dem häufig normalisierte Steckverbindungsstücke (RMN) und Gegensteckverbindungsstücke (RFN) verwendet werden, insbesondere für eine enterale Ernährungsleitung, wobei das Gegensteckverbindungsstück (RFI) wie das normalisierte Gegensteckverbindungsstück (RFN) einen Kopf (13; 14) hat, welcher einen konischen Eingangskanal (15; 16) festlegt und welcher ein Außengewinde (19; 20) ausbildet, und das Steckverbindungsstück (RMI) wie das normalisierte Steckverbindungsstück (RMN) einen überstehenden konischen Stutzen (1; 2) hat, welcher einen Eingangskanal (3; 4) festlegt, der von einem Kragenabschnitt (7; 8) umgeben ist, welcher um den Stutzen eine Aushöhlung (9; 10) festlegt und ein Innengewinde (11, 12) hat, wobei das Kopfende des Stutzens des Gegensteckverbindungsstücks (RFI) angepasst ist, in die Aushöhlung des Steckverbindungsstücks (RMI) einzudringen, wobei das Kopfende des Stutzens des Steckverbindungsstücks (RMI) angepasst ist, seitlich abdichtend in den Eingangskanal des Gegensteckverbindungsstücks (RFI) einzudringen, wobei die Gewinde der zwei Verbindungsstücke angepasst sind, zur Verriegelung des Zusammenbaus zusammenzuwirken, **dadurch gekennzeichnet, dass** die Verbindungsstücke (RMI, RFI) einen Eingangsdurchmesser und einen Durchmesser an der Gewindespitze aufweisen, die im Verhältnis zu den entsprechenden Durchmessern der normalisierten Verbindungsstücke (RMN, RFN) derart gewählt sind, dass der jeweilige Zusammenbau von einem Steckverbindungsstück (RMI) oder einem Gegensteckverbindungsstück (RFI) mit einem normalisierten Gegensteckverbindungsstück (RFN) oder Steckverbindungsstück (RMN) unterbunden wird, weil das Eindringen des Stutzens des Steckverbindungsstücks in den Eingangskanal des Gegensteckverbindungsstücks unmöglich ist oder weil das Eindringen durch Abstützung des Kopfes des Gegensteckverbindungsstücks gegen den Ringabschnitt des Steckverbindungsstücks aufgehalten wird.

2. Verbindungsstücke (RMI, RFI) gemäß Anspruch 1, wobei die Eingangskanäle (15; 16 - 3; 4) eine unterschiedliche Konizität von 6% (Luer-Konizität) haben.

3. Verbindungsstücke (RMI, RFI) gemäß Anspruch 2, wobei die Eingangskanäle (15; 16 - 3; 4) eine Konizität haben, die in den Bereich von 4% und 8-10% fallen.

4. Verbindungsstücke (RMI, RFI) gemäß einem der Ansprüche 1 bis 3, wobei die konischen Eingangskanäle (15; 16 - 3; 4) eine Länge von 6,5 mm haben.

5. Verbindungsstücke (RMI, RFI) gemäß einem der Ansprüche 1 bis 4, wobei die Gewinde Gewinde mit zwei Gewindegängen mit einem Abstand von 5 mm sind.

6. Verbindungsstücke (RMI, RFI) gemäß einem der Ansprüche 1 bis 5, wobei das Steckverbindungsstück (A) einen Eingangsdurchmesser von 3,2 mm und einen Durchmesser an der Gewindespitze von 5,8 mm hat und das Gegensteckverbindungsstück (B) einen Eingangsdurchmesser von 3,5 mm und einen Durchmesser an der Gewindespitze von 6,8 mm hat.

7. Verbindungsstücke (RMI, RFI) gemäß Anspruch 6, wobei das Steckverbindungsstück (A) einen Durchmesser am Gewindegrund von 7 mm hat.

8. Verbindungsstücke (RMI, RFI) gemäß Anspruch 6, wobei das Gegensteckverbindungsstück (B) einen Durchmesser am Gewindegrund von 5,6 mm hat.

9. Verbindungsstücke (RMI, RFI) gemäß einem der Ansprüche 1 bis 5, wobei das Steckverbindungsstück (C) einen Eingangsdurchmesser von 4,5 mm und einen Durchmesser an der Gewindespitze von 7,4 mm hat und das Gegensteckverbindungsstück (D) einen Eingangsdurchmesser von 4,8 mm und einen Durchmesser an der Gewindespitze von 8,2 mm hat.

10. Verbindungsstücke (RMI, RFI) gemäß Anspruch 9, wobei das Steckverbindungsstück (C) einen Durchmesser an dem Gewindegrund von 8,4 mm hat.

11. Verbindungsstücke (RMI, RFI) gemäß Anspruch 12, wobei das Gegenverbindungsstück (D) einen Durchmesser am Gewindegrund von 7,2 mm hat.

12. Verbindungsstücke (RMI, RFI) gemäß einem der Ansprüche 1 bis 11, welche hintere Kanäle (5, 6; 17, 18) haben, die ein Fixieren der Verbindungsstücke an Rohrleitungen ermöglichen.

13. Behälter, der mit einem Gegensteckverbindungsstück (RFI) ausgestattet ist, wie es in einem der Ansprüche 1 bis 11 definiert ist.

14. Behälter gemäß Anspruch 13 aus der Gruppe bestehend aus einer Tasche, einem Fläschchen, einer Flasche, einer Spritze.

15. Enterale Ernährungsversorgungsvorrichtung, die mit einem Gegensteckverbindungsstück (RFI) ausgestattet ist, wie es in einem der Ansprüche 1 bis 11 definiert ist.

16. Spritze mit einem Halm zur Aspiration von enteralen Ernährungsprodukten, wobei die Spritze mit einem Steckverbindungsstück (RMI) ausgestattet ist, das wie in einem der Ansprüche 1 bis 11 definiert ist.

17. Sonde mit einem Verbindungsende, das ein Gegensteckverbindungsstück (RFI) umfasst oder mit diesem vorgesehen ist, wie es in einem der Ansprüche 1 bis 11 definiert ist.

18. Rohrleitung mit einem Ende, das mit einem Steckverbindungsstück (RMI) gemäß einem der Ansprüche 1 bis 11 vorgesehen ist, und mit einem gegensätzlichen Ende, das mit einem Gegensteckverbindungsstück (RFI) gemäß einem der Ansprüche 1 bis 11 vorgesehen ist.

19. Verbindung mit einem Ende, das durch ein Gegensteckverbindungsstück (RFI) gemäß einem der Ansprüche 1 bis 11 ausgebildet wird, und mit einem anderen Ende, das angepasst ist, mit einem Behälter zur enteralen Versorgung verbunden zu werden.

20. Drei-Wege-Verbindung, wobei einer der Wege mit einem Steckverbindungsstück (RMI) gemäß einem der Ansprüche 1 bis 11 ausgestattet ist, wobei die zwei anderen Wege jeweils mit einem Gegensteckverbindungsstück (RFI) gemäß einem der Ansprüche 1 bis 11 ausgestattet sind.

21. Drei-Wege-Verbindung, wobei einer der Wege mit einem Gegensteckverbindungsstück (RFI) gemäß einem der Ansprüche 1 bis 11 ausgestattet ist, wobei die zwei anderen Wege jeweils mit einem Steckverbindungsstück (RMI) gemäß einem der Ansprüche 1 bis 11 ausgestattet sind.

22. Enterale Ernährungsleitung, die mit einem Steckverbindungsstück (RMI) und einem Gegensteckverbindungsstück (RFI) gemäß einem oder mehrerer der Ansprüche 1 bis 11 vorgesehen ist.

23. Verbindungsstücksatz, aufweisend normalisierte Verbindungsstücke (RMN, RFN) und Verbindungsstücke gemäß der Erfindung (RMI, RFI), die wie in der folgenden Tabelle definiert sind:
| DURCHMESSER (MM) | RMI | RFN | RFI | RMN |
|---|---|---|---|---|
| Eingangsdurchmesser | 3,2 | 4,270-4,315 | 3,5 | 3,925-4,027 |
| Durchmesser an Gewindegrund | 7 | 6,73 | 5,6 | 8 |
| Durchmesser an Gewindespitze | 5,8 | 7,83 | 6,8 | 7 |
| Eingangsdurchmesser | 4,5 | 4,270-4,315 | 4,8 | 3,925-4,027 |
| Durchmesser an Gewindegrund | 8,4 | 6,73 | 7,2 | 8 |
| Durchmesser an Gewindespitze | 7,4 | 7,83 | 8,2 | 7 |
